# Europäisches Patentamt

# European Patent Office

# Office européen des brevets

(19)

(11) Veröffentlichungsnummer : **0 415 214 B1**

(12) **EUROPÄISCHE PATENTSCHRIFT**

(45) Veröffentlichungstag der Patentschrift :
**02.02.94 Patentblatt 94/05**

(21) Anmeldenummer : **90115876.6**

(22) Anmeldetag : **18.08.90**

(51) Int. Cl.[5] : **C07C 67/317**, C07C 69/653,
C07C 231/12

(54) Verfahren zur Herstellung von alpha-Fluoracrylsäurederivaten.

(30) Priorität : **01.09.89 DE 3928990**

(43) Veröffentlichungstag der Anmeldung :
**06.03.91 Patentblatt 91/10**

(45) Bekanntmachung des Hinweises auf die
Patenterteilung :
**02.02.94 Patentblatt 94/05**

(84) Benannte Vertragsstaaten :
**BE DE FR GB IT**

(56) Entgegenhaltungen :
**EP-A- 0 132 681**

(56) Entgegenhaltungen :
**IZV. AKAD. NAUK SSSR; Band 1982, Nr. 3,
Seiten 654-657, 655, Zeilen 21-25; A.G. TALY-
BOV et al.: "Reaktion von Haloolefinen mit
Nitroniumtetrafluoroborat"
JOURNAL OF THE AMERICAN CHEMICAL SO-
CIETY, Band 76, Nr. 2, 1954, Seiten 479-481,
480, Spalte 2; A.L. HENNE et al.: "Ionization
constants of fluorinated acids. III. Unsaturated
acids"**

(73) Patentinhaber : **BAYER AG**
**D-51368 Leverkusen (DE)**

(72) Erfinder : **Gassen, Karl-Rudolf, Dr.**
**Auenweg 6a**
**D-5068 Odenthal (DE)**
Erfinder : **Marhold, Albrecht, Dr.**
**Carl-Duisberg-Strasse 329**
**D-5090 Leverkusen 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein vorteilhaftes Verfahren zur Herstellung von α-Fluoracrylsäurederivaten ausgehend von Dihalogenpropenen.

Derivate der α-Fluoracrylsäure sind vielseitige organische Zwischenprodukte und können insbesondere als Monomere zur Herstellung von Polymeren für optische Gläser, Lichtwellenleiter und deren Umhüllungen verwendet werden (siehe z.B. EP-A 0 128 517). Die bisher bekannten Verfahren zur Herstellung von α-Fluoracrylsäurederivaten sind nachteilig, weil sie viele Reaktionsstufen erfordern, schlechte Ausbeuten erbringen, schwierig zugängliche und deshalb teure toxische Chemikalien benötigen, deren Handhabung besondere sicherheitstechnische Aufwendungen erfordert, nicht reproduzierbar sind und/oder nur die Herstellung kleiner Mengen gestatten.

So ist es bisher bekannt, 2-Fluoracrylsäurefluorid aus Tetrafluoroxethan herzustellen (siehe EP-OS 0 148 490), welches nur mit großem technischem Aufwand zugänglich und sehr toxisch ist. Die Synthese ausgehend von Fluoressigsäure hat die gleichen Nachteile (siehe US-PS 3 075 002). Viele Reaktionsschritte benötigt ein Verfahren, das von 1,2-Dibrom-3-chlorpropan ausgeht (siehe Zh. Org. Khim 28 1173 (1987)). Eine elektrochemische Herstellungsmethode (siehe DE-A 3 704 915) erfordert großen apparativen Aufwand und liefert nicht immer reproduzierbare Resultate.

Es besteht deshalb das Bedürfnis nach einem einfach durchzuführenden und effektiven Verfahren zur Herstellung von α-Fluoracrylsäurederivaten in technischen Mengen.

Es wurde nun ein Verfahren zur Herstellung von α-Fluoracrylsäurederivaten gefunden, das dadurch gekennzeichnet ist, daß man

a) ein Dihalogenpropen der Formel (I)

$$CH_2=CHal^1-CH_2Hal^2 \qquad (I),$$

in der

Hal$^1$ und Hal$^2$ unabhängig voneinander für Chlor oder Brom stehen, mit einem wasserfreien Gemisch aus Salpetersäure und Fluorwasserstoffsäure zu einem 1-Nitro-2,2-fluorhalogen-3-halogenpropan der Formel (II) umsetzt

$$CH_2Hal^2-CFHal^1-CH_2NO_2 \qquad (II),$$

in der

Hal$^1$ und Hal$^2$ die bei Formel (I) angegebene Bedeutung haben,

b) dieses in Gegenwart einer starken Säure und wenig Wasser bei erhöhter Temperatur zu einer 2,2-Fluorhalogen-3-halogen-propionsäure der Formel (III) umwandelt

$$CH_2Hal^2-CFHal^1-COOH \qquad (III),$$

in der

Hal$^1$ und Hal$^2$ die bei Formel (I) angegebene Bedeutung haben,

c) diese in ein Derivat der Formel (IV) umwandelt

$$CH_2Hal^2-CFHal^1-CO-R \qquad (IV),$$

in der

Hal$^1$ und Hal$^2$ die bei Formel (I) angegebene Bedeutung haben und

R für OR$^1$ mit R$^1$ = gegebenenfalls substituiertem, geradkettigem, verzweigtem oder cyclischem C$_1$- bis C$_{18}$-Alkyl, gegebenenfalls substituiertem C$_6$- bis C$_{14}$-Aryl oder C$_6$- bis C$_{14}$-Heteroaryl

oder für NR$^2$R$^3$ mit R$^2$ und R$^3$ unabhängig voneinander = gegebenenfalls substituiertem, geradkettigem, verzweigtem oder cyclischem C$_1$- bis C$_{18}$-Alkyl oder wobei R$^2$ und R$^3$ gemeinsam mit dem dazwischen befindlichen Stickstoffatom einen 5- bis 7-gliedrigen Ring bilden

und

d) aus diesem durch Umsetzung mit einen Dehalogenierungsmittel ein α-Fluoracrylsäurederivat der Formel (V) erhält

$$CH_2=CF-CO-R \qquad (V),$$

in der

R die bei Formel (IV) angegebene Bedeutung hat.

In den Formeln (I) bis (IV) stehen Hal$^1$ und Hal$^2$ vorzugsweise für Chlor. Soweit es sich bei R$^1$, R$^2$ und R$^3$ um substituierte Reste handeln kann, kommen als Substituenten vorzugsweise Halogenatome in Frage, insbesondere Fluoratome.

Die Umsetzung des Dihalogenpropens der Formel (I) mit einem wasserfreien Gemisch aus Salpetersäure und Fluorwasserstoffsäure kann beispielsweise bei Temperaturen im Bereich von -50 bis +30°C erfolgen. Bevorzugte Temperaturen sind solche von -10 bis +20°C.

2

EP 0 415 214 B1

Das wasserfreie Gemisch aus Salpetersäure und Fluorwasserstoffsäure kann durch Vermischen der wasserfreien Komponenten, aber auch beispielsweise durch Zusatz von Schwefeltrioxid oder Chlorsulfonsäure zu wasserhaltigen Gemischen aus Salpetersäure und Fluorwasserstoffsäure erhalten werden.

Bezogen auf 1 Mol Dihalogenpropen der Formel (I) kann Salpetersäure beispielsweise in Mengen von 0,9 bis 10 Mol und Fluorwasserstoffsäure beispielsweise in Mengen von 2 bis 50 Mol eingesetzt werden. Vorzugsweise werden pro Mol Dihalogenpropen der Formel (I) 1 bis 2 Mol Salpetersäure und 5 bis 30 Mol Fluorwasserstoffsäure eingesetzt.

Die Umsetzung des Dihalogenpropens der Formel (I) mit dem Salpetersäure/Fluorwasserstoffsäure-Gemisch kann man z.B. durchführen, indem man das Säuregemisch vorlegt, das Dihalogenpropen langsam zufügt und nach beendeter Zugabe noch einige Zeit nachrührt. Die Aufarbeitung des Reaktionsgemisches kann z.B. erfolgen, indem man z.B. überschüssige Fluorwasserstoffsäure im Vakuum abdestilliert und so zurückgewinnt und den Rückstand ohne weitere Nachbehandlung, gegebenenfalls im selben Reaktionsgefäß für den nächsten Reaktionsschritt einsetzt. Man kann den Rückstand auch auf Eis gießen, mit Wasser waschen und nach Phasentrennung die noch etwas Wasser enthaltende organische Phase in den nächsten Reaktionsschritt einsetzen. Gegebenenfalls kann die organische Phase mit einem Trocknungsmittel, z.B. Natriumsulfat oder Calciumchlorid, behandelt werden.

Falls erwünscht, kann aus der so gereinigten und getrockneten Lösung durch Abziehen des Lösungsmittels und Destillation im Vakuum reines 1-Nitro-2,2-fluorhalogen-3-halogenpropan der Formel (II) erhalten werden. Bei einer derartigen Isolierung sind gegebenenfalls besondere Sicherheitsmaßnahmen zu ergreifen, da spontane Zersetzungen auftreten können.

Die Umwandlung zur entsprechenden 2,2-Fluorhalogen-3-halogen-propionsäure der Formel (III) erfordert eine Katalyse mit einer starken Säure. Als starke Säuren kommen insbesondere Schwefelsäure, Phosphorsäure, Salpetersäure, Chlorwasserstoffsäure und Bromwasserstoffsäure in Frage. Schwefelsäure ist bevorzugt. Wenn die starke Säure unter den anzuwendenden Temperaturen flüchtig ist muß man in geschlossenen Gefäßen unter Druck arbeiten. Die starke Säure kann beispielsweise in Mengen von 1 bis 100 Mol pro Mol der Verbindung der Formel (II) eingesetzt werden. Vorzugsweise liegt diese Menge bei 3 bis 50 Mol.

Die Umwandlung wird in Gegenwart von wenig Wasser durchgeführt, beispielsweise 0,9 bis 20 Mol Wasser pro Mol Verbindung der Formel (II). Vorzugsweise beträgt diese Menge 1 bis 10 Mol, besonders bevorzugt 3 bis 8 Mol. Das Wasser kann auf verschiedene Weise in das Reaktionsgemisch eingebracht werden, beispielsweise in Form einer wasserhaltigen, vorzugsweise über 90 gew.-%igen starken Säure und/oder in Form einer wasserhaltigen Verbindung der Formel (II).

Das benötigte Wasser kann auch als solches dem Reaktionsgemisch zugefügt werden. Zu beachten ist, daß die oben angegebenen Wassermengen sich auf die Summe des gesamten, zu Reaktionsbeginn im Reaktionsgemisch vorliegenden Wassers beziehen und während der Reaktion im allgemeinen kein Wasser mehr zugefügt wird.

Die Umwandlung kann z.B. bei Temperaturen im Bereich 50 bis 200°C durchgeführt werden. Vorzugsweise heizt man langsam oder stufenweise auf und führt die Reaktion z.B. während 1 bis 20 Stunden bei 50 bis 150°C zu Ende.

Die so erhaltene 2,2-Fluorhalogen-3-halogencarbonsäure der Formel (III) kann man aus dem Reaktionsgemisch beispielsweise isolieren, indem man dieses auf Eis gibt, dann mit einem oder mehreren inerten, mit Wasser nicht mischbaren Lösungsmitteln ein- oder mehrmals extrahiert, die vereinigten organischen Phasen trocknet, das oder die Lösungsmittel abzieht und den verbleibenden Rückstand destilliert, vorzugsweise im Vakuum.

Die anschließende Derivatisierung zu Verbindungen der Formel (IV) kann auf an sich bekannte Weise durchgeführt werden. Verbindungen der Formel (IV) mit R = OR$^1$ kann man durch Veresterung einer Säure der Formel (III) mit dem entsprechenden Alkohol HOR$^1$ erhalten.

Zur Herstellung von Verbindungen der Formel (IV) mit R = NR$^2$R$^3$ ist es erforderlich, die Säure der Formel (III) zunächst in das entsprechende Säurechlorid überzuführen, beispielsweise mit Thionylchlorid. Aus dem Säurechlorid kann man dann durch Umsetzung mit einem Amin der Formel HNR$^2$R$^3$ das Amid der Formel (IV) mit R = NR$^2$R$^3$ erhalten.

Auch bei der Herstellung von Estern der Formel (IV) mit R = OR$^1$ kann man zunächst die Säure der Formel (III) in das entsprechende Säurechlorid überführen und dann dieses mit einem Alkohol HOR$^1$ verestern. Vorzugsweise wählt man diesen Weg, wenn der Alkohol HOR$^1$ im sauren Milieu nicht beständig ist, sondern z.B. zu Umlagerungen neigt.

Bei den Formeln (IV) und (V) steht R vorzugsweise für $C_1$- bis $C_{18}$-Alkoxy oder -Fluoralkoxy oder eine Amidogruppe NR$^2$R$^3$, in der R$^2$ = R$^3$ ist und jeweils einen $C_1$- bis $C_{18}$-Alkyl- oder -Fluoralkylrest bedeutet oder für eine Amidogruppe NR$^2$R$^3$, wobei R$^2$ und R$^3$ gemeinsam mit dem dazwischen befindlichen Stickstoffatom einen 5-bis 7-gliedrigen Ring bilden.

3

Als Dehalogenierungsmittel für die Umsetzung einer Verbindung der Formel (IV) in eine Verbindung der Formel (V) kommen beispielsweise Zink, Magnesium oder Chrom(II)-chlorid in Gegenwart von protischen oder aprotischen Solventien in Frage. Die Dehalogenierung kann auch auf elektrochemische Weise durchgeführt werden. Bevorzugt ist Zink in Gegenwart von Wasser oder Diglyme. Geeignete Temperaturen für die Dehalogenierung sind beispielsweise solche von 0 bis 150°C.

Die Aufarbeitung kann so erfolgen, daß man das gebildete $\alpha$-Fluoracrylsäurederivat der Formel (V) aus dem Reaktionsgemisch abdestilliert, gegebenenfalls zusammen mit Wasser, die organischen Bestandteile des Destillats abtrennt und trocknet und durch eine erneute Destillation reinigt.

Die vorliegende Erfindung gestattet die Herstellung von $\alpha$-Fluoracrylsäurederivaten auf einfache Weise, in wenigen Reaktionsstufen, mit guten Ausbeuten, unter Verwendung von preiswerten, gut zugänglichen Chemikalien und in üblichen Apparaturen. Sie eignet sich deshalb besonders für die Herstellung von $\alpha$-Fluoracrylsäurederivaten in technischen Mengen.

Es ist ausgesprochen überraschend, daß das erfindungsgemäße Verfahren so einfach und gut durchführbar ist, denn bisher ist lediglich bekannt, daß man 1-Nitro-2,2-fluorhalogen-3-halogenpropan mit $NO_2BF_4$ in flüssigem Schwefeldioxid bei Temperaturen von -30 bis -40°C herstellen kann (siehe I zv. Akad. Nauk SSSR, Ser. Khim., Nr. 3, S. 654-657, März 1982). $NO_2BF_4$ ist ein teures, schwierig herzustellendes und nur in geringen Mengen verfügbares Reagenz, die Arbeitsweise in flüssigem Schwefeldioxid für ein technisches Verfahren äußerst aufwendig.

Da diese überraschenden Effekte besonders bei der ersten Stufe des erfindungsgemäßen Verfahrens zur Herstellung von $\alpha$-Fluoracrylsäurederivaten der Formel (V) auftreten, betrifft die vorliegende Erfindung auch ein Verfahren zur Herstellung von 1-Nitro-2,2-fluorhalogen-3-halogenpropan der Formel (II)

$$CH_2Hal^2\text{-}CFHal^1\text{-}CH_2NO_2 \qquad (II),$$

in der
Hal$^1$ und Hal$^2$ unabhängig voneinander für Chlor oder Brom stehen,
das dadurch gekennzeichnet ist, daß man ein Dihalogenpropen der Formel (I)

$$CH_2=CHal^1\text{-}CH_2Hal^2 \qquad (I),$$

in der
Hal$^1$ und Hal$^2$ die bei Formel (II) angegebene Bedeutung haben,
mit einem wasserfreien Gemisch aus Salpetersäure und Fluorwasserstoffsäure umsetzt.

## Beispiele

### Beispiel 1

Herstellung von 2,3-Dichlor-2-fluor-1-nitropropan

Zu einer Mischung aus 86 ml wasserfreier, 100 %iger Salpetersäure und 300 ml wasserfreier Fluorwasserstoffsäure wurden bei -10°C 111 g (1 Mol) 2,3-Dichlorpropen innerhalb von 3 Stunden zugetropft. Die Reaktion verlief leicht exotherm. Nach vollständigem Zutropfen wurde noch 1 Stunde bei -10°C nachgerührt. Anschließend wurde das Gemisch auf Eis gegeben und mit Dichlormethan extrahiert. Die organische Phase wurde mit Natriumhydrogencarbonat-Lösung gewaschen, über Natriumsulfat getrocknet und am Rotationsverdampfer eingeengt. Das Rohprodukt wurde im Vakuum einer Ölpumpe destilliert. Es wurden 115,8 g (= 63 % der Theorie) Produkt mit einem Siedepunkt von 48 bis 50°C bei 0,07 mbar und einem Brechungsindex $n_D^{20}$ von 1,4578 (bei 24°C) erhalten. Das Produkt wies eine Reinheit von 95,7 % auf.

Da die Gefahr besteht, daß das Destillationsgemisch sich zersetzt, wurde bei Wiederholungen der Herstellung von 2,3-Dichlor-2-fluor-1-nitropropan entsprechend Beispiel 2 verfahren.

### Beispiel 2

Herstellung von 2,3-Dichlor-2-fluor-1-nitropropan

Zu einer Mischung aus 970 ml wasserfreier, 100 %iger Salpetersäure und 3380 ml wasserfreier Fluorwasserstoffsäure wurden bei -10°C 1250 g (11,3 Mol) 2,3-Dichlorpropen innerhalb von 5 Stunden zugetropft. Die Reaktion verlief exotherm. Nach vollständigem Zutropfen wurde das Gemisch auf Eis gegeben. Die organische Phase wurde abgetrennt und mit viel Wasser gewaschen. Es wurden 1316 g feuchtes Produkt (= 66 % der Theorie) in einer Reinheit von 97 % erhalten. Der Wassergehalt des Produktes betrug 0,5 %.

Beispiel 3

Herstellung von 2,3-Dichlor-2-fluorpropionsäure

Eine Mischung aus 1170 g (6,65 Mol) 2,3-Dichlor-2-fluor-1-nitropropan (97 %ig), 1500 ml konzentrierter Schwefelsäure und 10 ml Wasser wurde langsam erwärmt, 2 Stunden bei 80°C, 4 Stunden bei 100°C und 6 Stunden bei 120°C gerührt. Der Ansatz wurde auf Eis gegeben, zuerst mit Chloroform und dann mit Diethylether extrahiert, die organischen Phasen vereinigt und über Natriumsulfat getrocknet. Anschließend wurden die Lösungsmittel am Rotationsverdampfer abgezogen und der Rückstand im Wasserstrahlvakuum destilliert. Es wurden 925 g (= 87 % der Theorie) Produkt mit einem Siedepunkt bei 18 mbar von 98 bis 101°C erhalten. Die Reinheit des Produktes betrug 92 %. Beim Kühlen der Vorlage erstarrte das Produkt.

Beispiel 4

Herstellung von 2,3-Dichlor-2-fluorpropionsäurechlorid

Eine Mischung aus 1096,5 g (6,8 Mol) 2,3-Dichlor-2-fluorpropionsäure (92 %ig), 2052 g (17,3 Mol) Thionylchlorid wurde langsam erwärmt und bei 78 bis 80°C so lange gerührt, bis die Gasentwicklung beendet war. Nach 16 Stunden war der Umsatz laut gaschromatographischer Analyse vollständig. Anschließend wurde überschüssiges Thionylchlorid und dann das Produkt über eine 60 cm lange Füllkörperkolonne abdestilliert. Es wurden 665 g (= 60 % der Theorie) Produkt mit einem Siedepunkt von 54°C bei 50 mbar erhalten. Die Reinheit des Produktes betrug 99,3 %.

Beispiel 5

Herstellung von 2,3-Dichlor-2-fluorpropionsäure-n-butylester

Zu einer Mischung aus 159 g (2,15 Mol) n-Butanol und 217 g (2,15 Mol) Triethylamin in 1200 ml trockenem Diethylether wurden bei 15 bis 20°C 350 g (1,95 Mol) 2,3-Dichlor-2-fluorpropionsäurechlorid zugetropft und anschließend noch 2 Stunden bei Raumtemperatur nachgerührt. Dann wurde auf 0°C abgekühlt, der ausfallende Feststoff abgesaugt und gut mit Diethylether gewaschen. Die organischen Phasen wurden vereinigt, einmal mit verdünnter Salzsäure gewaschen, über Natriumsulfat getrocknet, am Rotationsverdampfer eingeengt und anschließend destilliert. Es wurden 360 g (= 83 % der Theorie) Produkt mit einem Siedepunkt von 99 bis 101°C bei 26 mbar erhalten. Das Produkt wies eine Reinheit von 97 % auf.

Beispiel 6

Herstellung von $\alpha$-Fluoracrylsäure-n-butylestern

Zu einer Mischung aus 340 g mit 5 %iger $H_2SO_4$ frisch aktiviertem Zink, 700 ml Wasser und 1 ml konzentrierter Schwefelsäure wurden bei 110°C 338 g (1,56 Mol) 2,3-Dichlor-2-fluorpropionsäure-n-butylester getropft. Das Produkt destillierte azeotrop ab. Aus dem Destillat wurde die organische Phase abgetrennt und die wäßrige Phase gut mit Diethylether extrahiert. Die abgetrennte organische Phase und die Diethylether-Phasen wurden vereinigt, getrocknet, am Rotationsverdampfer eingeengt und anschließend destilliert. Die Ausbeute betrug 146,5 g (= 73 % der Theorie) Produkt mit einem Siedepunkt von 49 bis 51°C bei 24 mbar. Die Reinheit des Produktes betrug 98 %.

**Patentansprüche**

**1.** Verfahren zur Herstellung von $\alpha$-Fluoracrylsäurederivaten, dadurch gekennzeichnet, daß man
a) ein Dihalogenpropen der Formel (I)
$$CH_2=CHal^1-CH_2Hal^2 \qquad (I),$$
in der
$Hal^1$ und $Hal^2$ unabhängig voneinander für Chlor oder Brom stehen,
mit einem wasserfreien Gemisch aus Salpetersäure und Fluorwasserstoffsäure zu einem 1-Nitro-2,2-fluorhalogen-3-halogenpropan der Formel (II) umsetzt
$$CH_2Hal^2-CFHal^1-CH_2NO_2 \qquad (II),$$
in der
$Hal^1$ und $Hal^2$ die bei Formel (I) angegebene Bedeutung haben,
b) dieses in Gegenwart einer starken Säure, wenig Wasser und bei erhöhter Temperatur zu einer 2,2-Fluorhalogen-3-halogen-propionsäure der Formel (III) umwandelt

5

$$CH_2Hal^2\text{-}CFHal^1\text{-}COOH \qquad (III),$$

in der

Hal$^1$ und Hal$^2$ die bei Formel (I) angegebene Bedeutung haben,

c) diese in ein Derivat der Formel (IV) umwandelt

$$CH_2Hal^2\text{-}CFHal^1\text{-}CO\text{-}R \qquad (IV),$$

in der

Hal$^1$ und Hal$^2$ die bei Formel (I) angegebene Bedeutung haben und

R für OR$^1$ mit R$^1$ = gegebenenfalls substituiertem, geradkettigem, verzweigtem oder cyclischem C$_1$- bis C$_{18}$-Alkyl, gegebenenfalls substituiertem C$_6$-bis C$_{14}$-Aryl oder C$_6$- bis C$_{14}$-Heteroaryl

oder für NR$^2$R$^3$ mit R$^2$ und R$^3$ unabhängig voneinander = gegebenenfalls substituiertem, geradkettigem, verzweigtem oder cyclischem C$_1$- bis C$_{18}$-Alkyl oder wobei R$^2$ und R$^3$ gemeinsam mit dem dazwischen befindlichen Stickstoffatom einen 5- bis 7-gliedrigen Ring bilden

und

d) aus diesem durch Umsetzung mit einen Dehalogenierungsmittel ein α-Fluoracrylsäurederivat der Formel (V) erhält

$$CH_2=CF\text{-}CO\text{-}R \qquad (V),$$

in der

R die bei Formel (IV) angegebene Bedeutung hat.

2. Verfahren nach Anspruch 1, dadurch gekennzeichnet, daß in den Formeln (I) bis (IV) Hal$^1$ und Hal$^2$ für Chlor stehen.

3. Verfahren nach Ansprüchen 1 und 2, dadurch gekennzeichnet, daß man den Schritt a) bei Temperaturen im Bereich -50 bis +30°C durchführt.

4. Verfahren nach Ansprüchen 1 bis 3, dadurch gekennzeichnet, daß man im Schritt a) 0,9 bis 10 Mol Salpetersäure und 2 bis 50 Mol Fluorwasserstoffsäure, jeweils bezogen auf 1 Mol Dihalogenpropen der Formel (I), einsetzt.

5. Verfahren nach Ansprüchen 1 bis 4, dadurch gekennzeichnet, daß man den Schritt b) in Gegenwart von 1 bis 100 Mol einer starken Säure pro Mol der Verbindung der Formel (II) durchführt.

6. Verfahren nach Ansprüchen 1 bis 5, dadurch gekennzeichnet, daß man den Schritt b) in Gegenwart von 0,9 bis 20 Mol Wasser pro Mol der Verbindung der Formel (II) durchführt.

7. Verfahren nach Ansprüchen 1 bis 6, dadurch gekennzeichnet, daß man den Schritt b) bei Temperaturen im Bereich von 50 bis 200°C durchführt.

8. Verfahren zur Herstellung von 1-Nitro-2,2-fluorhalogen-3-halogenpropan der Formel (II)

$$CH_2Hal^2\text{-}CFHal^1\text{-}CH_2NO_2 \qquad (II),$$

in der

Hal$^1$ und Hal$^2$ unabhängig voneinander für Chlor oder Brom stehen,

dadurch gekennzeichnet, daß man ein Dihalogenpropen der Formel (I)

$$CH_2=CHal^1\text{-}CH_2Hal^2 \qquad (I),$$

in der

Hal$^1$ und Hal$^2$ die bei Formel (II) angegebene Bedeutung haben,

mit einem wasserfreien Gemisch aus Salpetersäure und Fluorwasserstoffsäure umsetzt.

## Claims

1. Process for the preparation of α-fluoroacrylic acid derivatives, characterized in that

a) a dihalogenopropene of the formula (I)

$$CH_2=CHal^1\text{-}CH_2Hal^2 \qquad (I),$$

in which

Hal$^1$ and Hal$^2$, independently of one another, represent chlorine or bromine,

is reacted with an anhydrous mixture of nitric acid and hydrofluoric acid to give a 1-nitro-2,2-fluorohalogeno-3-halogenopropane of the formula (II)

$$CH_2Hal^2\text{-}CFHal^1\text{-}CH_2NO_2 \qquad (II),$$

in which

Hal$^1$ and Hal$^2$      have the meaning given in formula (I),

b) which is converted in the presence of a strong acid and a little water at elevated temperature into a 2,2-fluorohalogeno-3-halogenopropionic acid of the formula (III)

$$CH_2Hal^2\text{-}CFHal^1\text{-}COOH \qquad (III),$$

in which

Hal$^1$ and Hal$^2$      have the meaning given in formula (I),

c) which is converted into a derivative of the formula (IV)

$$CH_2Hal^2\text{-}CFHal^1\text{-}CO\text{-}R \qquad (IV),$$

in which

Hal$^1$ and Hal$^2$      have the meaning given in formula (I) and

R      represents OR$^1$ where R$^1$ = optionally substituted, straight chain, branched or cyclic C$_1$-C$_{18}$-alkyl, optionally substituted C$_6$-C$_{14}$-aryl or C$_6$-C$_{14}$-heteroaryl or represents NR$^2$R$^3$ where R$^2$ and R$^3$, independently of one another, = optionally substituted, straight chain, branched or cyclic C$_1$-C$_{18}$-alkyl or where R$^2$ and R$^3$ form a 5- to 7-membered ring together with the nitrogen atom which is located between them

and

d) which is reacted with a dehalogenating agent to give an α-fluoroacrylic acid derivative of the formula (V)

$$CH_2=CF\text{-}CO\text{-}R \qquad (V),$$

in which

R      has the meaning given in formula (IV).

2. Process according to Claim 1, characterized in that, in the formulae (I) to (IV), Hal$^1$ and Hal$^2$ represent chlorine.

3. Process according to Claims 1 and 2, characterized in that step a) is carried out at temperatures in the range of from -50 to +30°C.

4. Process according to Claims 1 to 3, characterized in that in step a) 0.9 to 10 mol of nitric acid and 2 to 50 mol of hydrofluoric acid are used, relative in each case to 1 mol of dihalogenopropene of the formula (I).

5. Process according to Claims 1 to 4, characterized in that step b) is carried out in the presence of 1 to 100 mol of a strong acid per mole of the compound of the formula (II).

6. Process according to Claims 1 to 5, characterized in that step b) is carried out in the presence of 0.9 to 20 mol of water per mole of the compound of the formula (II).

7. Process according to Claims 1 to 6, characterized in that step b) is carried out at temperatures in the range of from 50 to 200°C.

8. Process for the preparation of 1-nitro-2,2-fluorohalogeno-3-halogenopropane of the formula (II)

$$CH_2Hal^2\text{-}CFHal^1\text{-}CH_2NO_2 \qquad (II)$$

in which

Hal$^1$ and Hal$^2$,      independently of one another, represent chlorine or bromine, characterized in that a dihalogenopropene of the formula (I)

$$CH_2=CHal^1\text{-}CH_2Hal^2 \qquad (I)$$

in which

Hal$^1$ and Hal$^2$      have the meaning given in formula (II), is reacted with an anhydrous mixture of nitric acid and hydrofluoric acid.

**Revendications**

1. Procédé de préparation de dérivés de l'acide α-fluoroacrylique, caractérisé en ce que l'on fait réagir
   a) un propène dihalogéné de formule (I)

$$CH_2=CHal^1-CH_2Hal^2 \qquad (I),$$

dans laquelle

$Hal^1$ et $Hal^2$ représentent, indépendamment l'un de l'autre, un atome de chlore ou de brome, avec un mélange anhydre d'acide nitrique et d'acide fluorhydrique pour obtenir un 1-nitro-2,2-fluorohalogéno-3-halogénopropane de formule (II)

$$CH_2Hal^2-CFHal^1-CH_2NO_2 \qquad (II),$$

dans laquelle

$Hal^1$ et $Hal^2$ ont la même signification que dans la formule (I),

b) que l'on transforme ce produit en présence d'un acide fort et d'un peu d'eau à température élevée en un acide 2,2-fluorohalogéno-3-halogénopropionique de formule (III)

$$CH_2Hal^2-CFHal^1-COOH \qquad (III),$$

dans laquelle $Hal^1$ et $Hal^2$ ont la même signification que dans la formule (I),

c) que l'on transforme ce composé en un dérivé de formule (IV)

$$CH_2Hal^2-CFHal^1-CO-R \qquad (IV),$$

dans laquelle

$Hal^1$ et $Hal^2$ ont la même signification que dans la formule (I) et

R représente $OR^1$ avec $R^1$ = à un radical alkyle en $C_1$-$C_{18}$ cyclique, linéaire ou ramifié et éventuellement substitué, un radical aryle en $C_6$-$C_{14}$ éventuellement substitué ou un radical hétéroaryle en $C_6$-$C_{14}$

ou bien R représente $NR^2R^3$ où $R^2$ et $R^3$ représentent, indépendamment l'un de l'autre, un radical alkyle en $C_1$-$C_{18}$ linéaire, ramifié ou cyclique éventuellement substitué ou dans lequel $R^2$ et $R^3$ forment avec l'atome d'azote intermédiaire un cycle à 5 à 7 éléments

et

d) fait réagir ce produit avec un agent de déshalogénation pour obtenir un dérivé de l'acide $\alpha$-fluoroacrylique de formule (V)

$$CH_2=CF-CO-R \qquad (V),$$

dans laquelle

R a la signification indiquée pour la formule (IV).

2. Procédé selon la revendication 1, caractérisé en ce que, dans les formules (I) à (IV), $Hal^1$ et $Hal^2$ représentent chacun un atome de chlore.

3. Procédé selon les revendications 1 et 2, caractérisé en ce que l'on effectue l'étape a) à des températures comprises dans l'intervalle de -50 à +30°C.

4. Procédé selon les revendications 1 à 3, caractérisé en ce que l'on utilise pour l'étape a) de 0,9 à 10 moles d'acide nitrique et de 2 à 50 moles d'acide fluorhydrique rapportées chaque fois à une mole de propène dihalogéné de formule (I).

5. Procédé selon les revendications 1 à 4, caractérisé en ce que l'on effectue l'étape b) en présence de 1 à 100 moles d'un acide fort pour 1 mole du composé de formule (II).

6. Procédé selon les revendications 1 à 5, caractérisé en ce que l'on effectue l'étape b) en présence de 0,9 à 20 moles d'eau pour 1 mole du composé de formule (II).

7. Procédé selon les revendications 1 à 6, caractérisé en ce que l'on effectue l'étape b) à des températures comprises dans l'intervalle de 50 à 200°C.

8. Procédé de préparation du 1-nitro-2,2-fluorohalogéno-3-halogénopropane de formule (II)

$$CH_2Hal^2-CFHal^1-CH_2NO_2 \qquad (II),$$

dans laquelle

$Hal^1$ et $Hal^2$ représentent, indépendamment l'un de l'autre, un atome de chlore ou de brome, caractérisé en ce que l'on fait réagir un propène dihalogéné de formule (I)

$$CH_2=CHal^1-CH_2Hal^2 \qquad (I),$$

dans laquelle

$Hal^1$ et $Hal^2$ ont la signification indiquée pour la formule (II) avec un mélange anhydre d'acide nitrique et d'acide fluorhydrique.